Europäisches Patentamt

European Patent Office

Office européen des brevets

(11) Veröffentlichungsnummer: **0 195 084**
**B1**

(12) **EUROPÄISCHE PATENTSCHRIFT**

(45) Veröffentlichungstag der Patentschrift: 23.05.90

(21) Anmeldenummer: 84904229.6

(22) Anmeldetag: 21.09.84

(86) Internationale Anmeldenummer:
PCT/SU84/00052

(87) Internationale Veröffentlichungsnummer:
WO 86/01723 27.03.86 Gazette 86/07

(51) Int. Cl.⁵: **A 61 K 37/66,** A 61 K 35/14,
C 07 K 3/24

(54) REKTIFIZIERUNGSVERFAHREN MENSCHLICHEN LEUKOZYTEN-INTERFERONS.

(43) Veröffentlichungstag der Anmeldung:
24.09.86 Patentblatt 86/39

(45) Bekanntmachung des Hinweises auf die
Patenterteilung:
23.05.90 Patentblatt 90/21

(84) Benannte Vertragsstaaten:
BE CH DE FR LI SE

(56) Entgegenhaltungen:
FR-A-2 351 665
US-A-3 981 991
US-A-4 017 600

SCIENCE, Band 202, 22. Dezember 1978, Seiten
1289-1290, AAAS; M. RUBINSTEIN et al.:
"Human leukocyte interferon purified to
homogeneity"

(73) Patentinhaber: VSESOJUZNY NAUCHNO-
ISSLEDOVATELSKY INSTITUT OSOBO
CHISTYKH BIOPREPARATOV
ul. Pudozhskaya 7
Leningrad, 197011 (SU)

(72) Erfinder: PIVOVAROV, Anatoly Mikhailovich
pr. K. Marxa, 7-15a
Leningrad, 194175 (SU)
Erfinder: KOROBITSYN, Leonid Pavlovich
ul. Kompozitorov, 11/1-8
Leningrad, 194355 (SU)
Erfinder: PASECHNIK, Vladimir Artemovich
pr. M. Toreza, 39-3-80
Leningrad, 194223 (SU)
Erfinder: ZEROV, Jury Petrovich
ul. Kompozitorov, 5-28
Leningrad, 194356 (SU)
Erfinder: SHATININA, Svetlana Vladimirovna
ul. Brjusovskaya, 26-53
Leningrad, 195271 (SU)
Erfinder: PASHKOVSKY, Alexandr Kolosovich
ul. Bestuzhevskaya, 15-19
Leningrad, 195271 (SU)
Erfinder: SCHEGLOVA, Natalya Anatolievna
ul. Kartashikhina, 19-213
Leningrad, 199151 (SU)

Courier Press, Leamington Spa, England.

EP 0 195 084 B1

**EP  0 195 084  B1**

(72) Erfinder: **SHARAPOV, Anatoly Platonovich**
**poselok Zelenogorsky, 33-9 Kalininskaya obl.**
**V. Volochek, 171130 (SU)**
Erfinder: **UDOVCHENKO, Vladimir Alexeevich**
**ul. 4 Mariinoi Roschi, 4-110**
**Moscow, 129272 (SU)**

(74) Vertreter: **von Füner, Alexander, Dr. et al**
**Patentanwälte v. Füner, Ebbinghaus, Finck**
**Mariahilfplatz 2 & 3**
**D-8000 München 90 (DE)**

**Beschreibung**

Gebiet der Technik

Die Erfindung bezieht sich auf das Gebiet der Biochemie, insbesondere auf Verfahren zur Reinigung des menschlichen leukozytären Interferons.

Vorheriger Stand der Technik

Es ist ein Verfahren zur Reinigung des menschlichen leukozytären Interferons bekannt (siehe Cantell K., Hirvonen S., Interferon System, A Current Review to 1978, Ed. S. Baron, F. Dianzani, 1977, Nr. 35, S. 138), das die Ausfällung der Eiweiße mit einer Kaliumthiozyanatlösung, die Abtrennung des Eiweißniederschlags und die Isolierung des Endproduktes durch Vielstufenextraktion mit Ethanol im sauren Medium vorsieht.

Das angegebene Verfahren gestattet es, das Endprodukt mit einer spezifischen Aktivität bis 10⁶ IE/mg Eiweiß zu erhalten.

Jedoch kann das Endprodukt mit der angegebenen spezifischen Aktivität nur beim Einhalten eines pH-Wertes des Mediums mit der Genauigkeit von 0,01 erhalten werden, was die Verwendung dieses Verfahrens unter großtechnischen Verhältnissen äußerst erschwert.

Außerdem ist das angegebene Verfahren arbeitsaufwendig, weil es eine Siebenstufenextraktion mit Ethanol im sauren Medium vorsieht.

Es ist auch ein Verfahren zur Reinigung von menschlichem leukozytärem Interferon bekannt (siehe C. B. Anfinsen, Proc. Nat. Acad. Sci., "The process of making interferon", 1974, 71, 3139), das die Ausfällung der nativen Eiweiße mit Ammoniumsulfat, die Abtrennung des Eiweißniederschlags, die Auflösung des abgetrennten Niederschlags und die Gelfiltration des Endproduktes vorsieht.

Jedoch gestattet es das angegebene Verfahren nicht, das Endprodukt in einer hohen Ausbeute und mit einem hohen Reinheitsgrad zu erhalten, was auf die Aggregation des Interferons mit schweren Eiweißen im Laufe der Gelfiltration zurückzuführen ist.

Offenbarung der Erfindung

Der vorliegenden Erfindung liegt die Aufgabe zugrunde, im Verfahren zur Reinigung des menschlichen leukozytären Interferons die Bedingungen der Gelfiltration auf die Weise auszuwählen, daß die Ausbeute und der Reinheitsgrad des Endproduktes erhöht werden.

Die gestallte Aufgabe wird dadurch gelöst, daß im erfindungsgemäßen Verfahren zur Reinigung des menschlichen leukozytären Interferons, das die Ausfällung der nativen Eiweiße mit Ammoniumsulfat, die Abtrennung des Eiweißniederschlags, die Auflösung des abgetrennten Niederschlags und die Gelfiltration des Endproduktes vorsieht, erfindungsgemäß der Niederschlag im Puffer mit einem pH-Wert von 2,5 bis 9,0, der ein desaggregierendes Reagens enthält, gelöst wird und vor der Gelfiltration auf die Kolonne derselbe Puffer in einer Menge von 3 bis 5% vom Kolonnenvolumen aufgetragen wird.

Die Durchführung der Stufen der Niederschlagauflösung und der Gelfiltration des Endproduktes in Gegenwart des desaggregierenden Reagens gestattet es, die Eiweißkomplexe zu zerstören, was zur Erhöhung des Reinheitsgrades und der Ausbeute an Endprodukt führt.

In Medien mit einem pH-Wert über 9,0 und unter 2,5 ist Interferon instabil.

Im Falle, wo auf die Kolonne vor der Gefiltration der Puffer, der für die Eiweißauflösung verwendet wird, in einer Menge unter 3% vom Kolonnenvolumen aufgetragen wird, wird im Laufe der Gelfiltration eine teilweise Reduktion der Eiweißkomplexe nachgewiesen, was zur Herabsetzung des Reinigungsgrades und zu Verlusten an Endprodukt führt.

Wenn die Menge des auf die Kolonne aufgetragenen Puffers über 5% vom Kolonnenvolumen beträgt, wird das desaggregierende Reagens im Bestand des Endproduktes beobachtet.

Es ist zweckmäßig, als desaggregierendes Reagens Harnstoff in einer Konzentration im Puffer von 2,0 bis 8,0 Mol zu verwenden.

Bei einer Verminderung des Harnstoffgehalts im Puffer unter 2,0 Mol kommt es zu keiner vollständigen Zerstörung der Eiweißkomplexe, was den Reinigungsgrad und die Ausbeute an Endprodukt herabsetzt.

Bei einer Vergrößerung des Harnstoffgehalts im Puffer über 8,0 Mol fällt Harnstoff aus.

In einer anderen Variante der Ausführung der Erfindung kann als das desaggregierende Reagens Guanidinhydrochlorid in einer Konzentration im Puffer von 1,0 bis 6,0 Mol verwendet werden.

Bei einer Verminderung des Guanidinhydrochloridgehalts im Puffer unter 1,0 Mol erfolgt keine vollständige Zerstörung der Eiweißkomplexe, was den Reinigungsgrad und die Ausbeute an Endprodukt herabsetzt.

Bei einer Vergrößerung des Guanidinhydrochloridgehalts im Puffer über 6,0 Mol fallt Guanidinhydrochlorid aus.

Beste Ausführungsvariante der Erfindung

Das erfindungsgemäße Verfahren wird wie folgt durchgeführt.

Dem nativen Interferon wird Ammoniumsulfat bis zu einer Konzentration von mindestens 70% der Sättigung zugegeben, wodurch das leukozytäre Interferon und andere Eiweiße gefällt werden. Der Niederschlag wird durch Zentrifugieren abgetrennt. Der abgetrennte Eiweißniederschlag wird in einer 0,1 molaren Pufferlösung mit einem pH-Wert von 2,5 bis 9,0 aufgelöst, die ein desaggregierendes Reagens enthält.

Auf die Kolonne mit Gel wird vor der Gelfiltration der erhaltenen Eiweißlösung derselbe Puffer, der das desaggregierende Reagens enthält, in einer Menge von 3 bis 5% vom Kolonnenvolumen aufgetragen.

Die Gelfiltration wird am Gel mit dem Trennungsbereich der Eiweiße nach der Molekularmasse von 5 bis 70 kD durchgeführt.

Als Gel wird Ultrogel (LKB) oder ein anderes Gel verwendet, das gegenüber der Einwirkung der desaggregierenden Reagenzien verhältnismäßig stabil ist und es ermöglicht, eine effektive Trennung der Eiweiße mit Molekularmassen von 5 bis 70 kD durchzuführen.

Interferon wird aus der Kolonne mit einer 0,1 bis 0,2 molaren Pufferlösung bei einem pH-Wert von 2,5 bis 9,0 eluiert.

Der Gehalt der Pufferlösung und der Kolonne mit dem Gel an desaggregierendem Reagens wird je nach den Besonderheiten des verwendbaren Rohstoffes ausgewählt.

Beispiel 1

Zu 8810 ml nativem Interferon (2000 IE/ml, 2 mg/ml Eiweiß) setzt man 4405 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 2,5 gelöst, der Harnstoff in einer Konzentration in der Lösung von 8 Mol enthielt. Die mit Ultragel gefüllte Kolonne K 100/100 wurde mit 0,1 molarem Phosphatpuffer mit einem pH-Wert von 2,5 ausgeglichen und darauf wurden 240 ml (3% vom Kolonnenvolumen) einer 8 molaren Harnstofflösung in demselben Puffer aufgetragen. Unmittelbar danach wurde auf die Kolonne die Interferonlösung aufgetragen und mit 0,1 molarem Phosphatpuffer mit einem pH-Wert von 2,5 eluiert. Das Volumen der gesammelten aktiven Fraktion betrug 700 ml. Das gereinigte Präparat wurde durch die spezifische Aktivität von 500.000 IE/mg Eiweiß, die Volumenaktivität von 100.000 IE/ml und die Gesamtaktivität von $7 \cdot 10^7$ IE gekennzeichnet.

Beispiel 2

Zu 8585 ml nativem Interferon (3.000 IE/ml, 2 mg/ml Eiweiß) setzt man 4292 g Ammoniumsulfat zu. Der ausgefallene Eiweißniederschlag wurde durch Zentrifugieren abgetrennt. Der abgetrennte Eiweißniederschlag wurde in einem 0,1 molarem Phosphatpuffer mit einem pH-Wert von 6,0 gelöst, der Harnstoff in einer Konzentration in der Lösung von 6 Mol enthielt. Die mit Ultragel gefüllte Kolonne K 100/100 wurde mit 0,1 molarem Phosphatpuffer mit einem pH-Wert von 6,0 ausgeglichen und darauf wurden 400 ml (5% vom Kolonnenvolumen) einer 6 molaren Harnstofflösung in demselben Puffer aufgetragen. Unmittelbar danach wurde auf die Kolonne Interferonlösung aufgetragen und mit 0,1 molarem Phosphatpuffer mit einem pH-Wert von 6,0 eluiert. Das Volumen der gesammelten aktiven Fraktion betrug 1360 ml. Das gereinigte Präparat wurde durch die spezifische Aktivität von 300.000 IE/mg Eiweiß, die Volumenaktivität von 75.000 IE/ml und die Gesamtaktivität von $9,7 \cdot 10^7$ IE gekennzeichnet.

Beispiel 3

Zu 8270 ml nativem Interferon (2480 IE/ml, 1 mg/ml Eiweiß) setzt man 4135 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 9,0 gelöst, der Harnstoff in einer Konzentration in der Lösung von 4 Mol enthielt. Die mit Ultrogel gefüllte Kolonne K 100/100 wurde mit 0,1 molarem Phosphatpuffer mit einem pH-Wert von 9,0 ausgeglichen und darauf wurden 320 ml (4% vom Kolonnenvolumen) einer 4 molaren Harnstofflösung in demselben Puffer aufgetragen. Unmittelbar danach wurde auf die Kolonne Interferonlösung aufgetragen und mit 0,1 molaren Phosphatpuffer mit einem pH-Wert von 9,0 eluiert. Das Volumen der gesammelten aktiven Fraktion betrug 900 ml. Das gereinigte Präparat wurde durch die spezifische Aktivität von 50.000 IE/mg Eiweiß, die Volumenaktivität von 20.000 IE/ml und die Gesamtaktivität von $1,8 \cdot 10^7$ IE gekennzeichnet.

Beispiel 4

Zu 8980 ml nativem Interferon (4960 IE/ml, 3 mg/ml Eiweiß) wurden 4490 g Ammoniumsulfat zugesetzt. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 7,0 gelöst, der Harnstoff in einer Konzentration in der Lösung von 2 Mol enthielt. Die mit Ultrogel gefüllte Kolonne K 100/100 wurde mit einem 0,1 molaren Phosphatpuffer mit einem pH-Wert von 7,0 ausgeglichen und darauf wurden 300 ml (3,75% vom Kolonnenvolumen) einer 2 molaren Harnstofflösung in demselben Puffer aufgetragen. Unmittelbar danach wurde auf die Kolonne die Interferonlösung aufgetragen und mit 0,1 molarem Phosphatpuffer mit einem pH-Wert von 7,0 eluiert. Das Volumen der gesammelten aktiven Fraktion betrug 1280 ml. Das gereinigte Präparat wurde durch die spezifische Aktivität von 400.000 IE/mg Eiweiß, die Volumenaktivität von 70.000 IE/ml und die Gesamtaktivität von $9,4 \cdot 10^7$ IE gekennzeichnet.

Beispiel 5

Zu 750 ml nativem Interferon (3500 IE/ml, 3,3 mg/ml Eiweiß) setzt man 308 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 7,0 gelöst, der Guanidinhydrochlorid in einer Konzentration in der Lösung von 1 Mol enthielt. Die mit Gel gefüllte Kolonne K 50/100 wurde mit einem 0,1 molaren Phosphatpuffer mit einem pH-Wert von 7,0 ausgeglichen und darauf wurden 80 ml (4% vom Kolonnenvolumen) einer 1 molaren Guanidinhydrochloridlösung in demselben Puffer aufgetragen. Unmittelbar danach wurde auf die Kolonne die Interferonlösung aufgetragen und mit 0,1 molarem Phosphatpuffer mit einem pH-Wert von 7,0 eluiert. Das Volumen der gesammelten aktiven Fraktion betrug 200 ml. Das gereinigte Präparat wurde durch die spezifische Aktivität von 30.000 IE/mg Eiweiß, die Volumenaktivität von 15.000 IE/ml und die Gesamtaktivität von $6 \cdot 10^6$ IE gekennzeichnet.

Beispiel 6

Zu 760 ml nativem Inteferon (3500 IE/ml, 3,3 mg/ml Eiweiß) setzt man 310 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 7,0 gelöst, der Guanidinhydrochlorid in einer Konzentration in der Lösung von 6 Mol enthielt. Die mit Ultrogel gefüllte Kolonne K 50/100 wurde mit einem 0,1 molaren Phosphatpuffer mit einem pH-Wert von 7,0 ausgeglichen und darauf wurden 60 ml (3% vom Kolonnenvolumen) einer 6 molaren Guanidinhydrochloridlösung in demselben Puffer aufgetragen. Unmittelbar danach wurde auf die Kolonne die Interferonlösung aufgetragen und mit 0,1 molarem Phosphatpuffer mit einem pH-Wert von 7,0 eluiert. Das Volumen der gesammelten aktiven Fraktion betrug 220 ml. Das gereinigte Präparat wurde durch die spezifische Aktivität von 35.000 IE/mg Eiweiß, die Volumenaktivität von 15.000 IE/ml und die Gesamtaktivität von $6,2 \cdot 10^6$ IE gekennzeichnet.

Beispiel 7

Zu 935 ml nativem Interferon (2000 IE/ml, 2,0 mg/ml Eiweiß) setzt man 466 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 3,0 gelöst, der Guanidinhydrochlorid in einer Konzentration in der Lösung von 3 Mol enthielt. Die mit Ultrogel gefüllte Kolonne K 50/100 wurde mit einem 0,1 molaren Phosphatpuffer mit einem pH-Wert von 3,0 ausgeglichen, und darauf wurden 60 ml (3% vom Kolonnenvolumen) einer 3 molaren Guanidinhydrochloridlösung in demselben Puffer aufgetragen. Unmittelbar danach wurde auf die Kolonne die Interferonlösung aufgetragen und mit einem 0,1 molaren Phosphatpuffer mit einem pH-Wert von 3,0 eluiert. Das Volumen der gesammelten aktiven Fraktion betrug 240 ml. Das gereinigte Präparat wurde durch die spezifische Aktivität von 96.000 IE/mg Eiweiß, die Volumenaktivität von 32.000 IE/ml und die Gesamtaktivität von $7,7 \cdot 10^6$ IE gekennzeichnet.

Beispiel 8

Zu 1230 ml nativem Interferon (7000 IE/ml, 3,5 mg/ml Eiweiß) setzt man 613 g Ammoniumsulfat zu. Der ausgefallene Eiweißniederschlag wurde durch Zentrifugieren abgetrennt. Der abgetrennte Eiweißniederschlag wurde in einem 0,1 molaren Phosphatpuffer mit einem pH-Wert von 8,0 gelöst, der Guanidinhydrochlorid in einer Konzentration in der Lösung von 4 Mol enthielt. Die mit Ultrogel gefüllte Kolonne K 50/100 wurde mit einem 0,1 molaren Phosphatpuffer mit einem pH-Wert von 8,0 ausgeglichen und darauf wurden 60 ml (3% vom Kolonnenvolumen) einer 4 molaren Guanidinhydrochloridlösung in demselben Puffer aufgetragen. Unmittelbar danach wurde auf die Kolonne die Interferonlösung aufgetragen und mit einem 0,1 molarem Phosphatpuffer mit einem pH-Wert von 8,0 eluiert. Das Volumen der gesammelten aktiven Fraktion betrug 250 ml. Das gereinigte Präparat wurde durch die spezifische Aktivität von 20.000 IE/mg Eiweiß, die Volumenaktivität von 10.000 IE/ml und die Gesamtaktivität von $5 \cdot 10^6$ IE gekennzeichnet.

Industrielle Anwendbarkeit

Am erfolgreichsten kann die vorliegende Erfindung in der pharmazeutischen Industrie zur Reinigung von menschlichem leukozytärem Interferon verwendet werden, das nach einem beliebigen Verfahren aus den Leukozyten sowohl von Spender- als auch Kadaverblutes erhalten wird.

**Patentansprüche**

1. Verfahren zur Reinigung natürlichen menschlichen leukozytären Interferons, das die Ausfällung der natürlichen Eiweiße mit Ammoniumsulfat, die Abtrennung des Eiweißniederschlags, die Auflösung des abgetrennten Niederschlags und die Gelfiltration des Endproduktes vorsieht, dadurch gekennzeichnet, daß man den Niederschlag in einem Puffer bei einem pH-Wert von 2,5 bis 9,0 löst, der ein desaggregierendes Agens enthält, und vor der Gelfiltration auf die Kolonne denselben Puffer in einer Menge von 3 bis 5% vom Kolonnenvolumen aufträgt.

2. Verfahren nach Anspruch 1, dadurch gekennzeichnet, daß man als desaggregierendes Agens Harnstoff in einer Konzentration im Puffer von 2,0 bis 8,0 Mol verwendet.

3. Verfahren nach Anspruch 1, dadurch gekennzeichnet, daß man als desaggregierendes Agens Guanidinhydrochlorid in einer Konzentration im Puffer von 1,0 bis 6,0 Mol verwendet.

**Revendications**

1. Procédé pour la purification d'interféron leucocitaire humain, où l'on prévoit la précipitation de l'albumine naturelle au sulfate d'ammonium, la séparation du précipité d'albumine, la dissolution du précipité séparé et la filtration sur gel du produit final, caractérisé en ce que l'on dissout le précipité dans un tampon à un pH de 2,5 à 9, lequel contient un agent de désagrégation et on applique, avant la filtration sur gel, sur la colonne, le même tampon en une quantité de 3 à 5% du volume de la colonne.

2. Procédé selon la revendication 1, caractérisé en ce qu'on utilise, en tant qu'agent de désagrégation, de l'urée, à une concentration dans le tampon, de 2,0 à 8,0 moles.

3. Procédé selon la revendication 1, caractérisé en ce qu'on utilise, en tant qu'agent de désagrégation, du chlorhydrate de guanidine à une concentration, dans le tampon, de 1,0 à 6,0 moles.

## Claims

1. A method for purifying natural human leukocyte interferon comprising the ammonium sulphate precipitation of natural proteins, the separation of the protein precipitate, the dissolution of the separated precipitate and the gel filtration of the end product, characterized in that the precipitate is dissolved in a buffer having a pH of from 2.5 to 9.0 and containing a disaggregating agent, and the same buffer is charged to the column before the gel filtration in an amount of 3 to 5% of the volume of the column.

2. A method according to Claim 1, characterized in that as the disaggregating agent urea is used in a concentration of 2.0 to 8.0 Mol in the buffer.

3. A method according to Claim 1, characterized in that as the disaggregating agent guanidine hydrochloride is used in a concentration of 1.0 to 6.0 Mol in the buffer.